# EUROPEAN PATENT APPLICATION

(11) **EP 3 357 531 A1**
(43) Date of publication of application: **08.08.2018**
(21) Application number: 16850063.5
(22) Date of filing: 15.04.2016
(51) Int. Cl.: A61M 16/20

(54) **SUPPORTING FRAME FOR MOUNTING ONE-WAY MEMBRANE INSIDE RESPIRATORY VALVE**

(30) Priority: 29.09.2015 CN 201510632186
(71) Applicant: Beijing Aeonmed Co., Ltd., Beijing 100070 (CN)
(72) Inventor: MA, Yingdan, Beijing 100070 (CN); SHEN, Youfang, Beijing 100070 (CN)
(74) Representative: Hanna Moore + Curley
(86) International application number: PCT/CN2016/079344
(87) International publication number: WO 2017/054437

(57) **Abstract**

Disclosed is a supporting frame for mounting a one-way membrane inside a respiratory valve, wherein the supporting frame (8) is arranged on the radical cross-section of the air inlet channel (3) inside the respiratory valve and is an integrated structure together with the air inlet channel (3), a mounting hole (801) for mounting the one-way membrane (2) is arranged in the centre of the supporting frame (8), and several air holes (802) are circumferentially distributed along the edge of the mounting hole (801). The supporting frame simplifies the fixing components needed by the support through designing same and the valve body of the respiratory valve as an integrated structure, makes the internal structure of the respiratory valve more compact, and avoids the risk of having the support dropping out.

## Description

### TECHNICAL FIELD

The present invention relates to the technical field of respiratory valves, and in particular to a supporting frame for mounting a one-way membrane inside a respiratory valve.

### BACKGROUND OF THE INVENTION

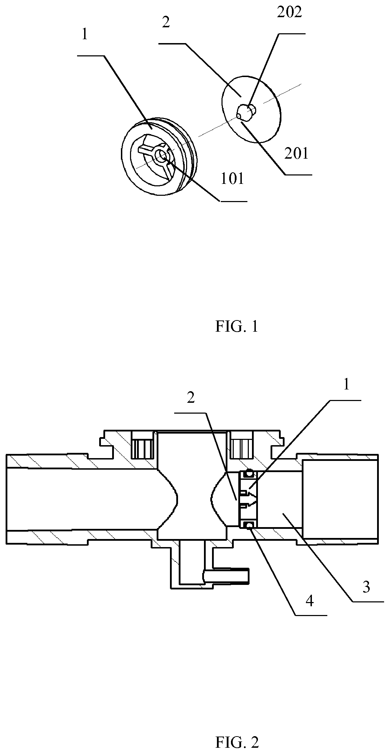

As shown in Fig. 1, in an existing respiratory valve, a one-way membrane 2 is generally mounted at the end of an air inlet channel to prevent exhaled air of a patient from flowing back and further contaminating a machine. Since a size of the respiratory valve is small, in order to facilitate the mounting of the one-way membrane 2, typically a support 1 is separately provided. First of all, the one-way membrane 2 is externally mounted on the support 1. Specifically, a conic small tip 201 provided on the one-way membrane 2 protrudes into a mounting hole 101 at the center of the support 1. Then, a conic large tip 202 provided on the one-way membrane 2 is pressed hard to pass through the mounting hole 101 as well. And then, the support 1 is pushed to a predefined position inside the respiratory valve along an air inlet channel 3.

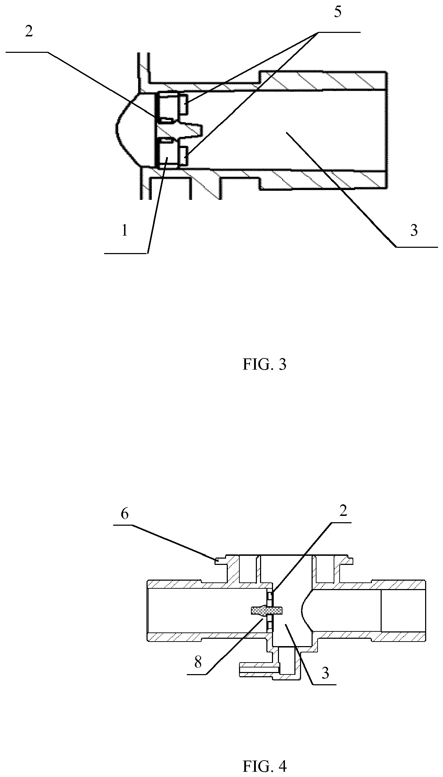

At this point, in order to prevent the support 1 from falling out of the air inlet channel 3, as shown in Fig. 2, a sealing ring 4 is usually mounted on the support 1, which is fixed with an amount of compression of the sealing ring 4; or a manner of arrangement as shown in Fig. 3 is used, that is, protrusions 5 are designed on an inner wall of the air inlet channel 3, and the support 1 is clamped after being pushed into the respiratory valve with plastic deformation. However, after being pushed into the air inlet channel 3, the support 1 is quite difficult to be taken out without special tools. Therefore, the above two manners of mounting the one-way membrane merely solve the problem of first-time mounting of the membrane and are difficult to perform membrane replacement operations, which in turn reduces a working efficiency.

### SUMMARY OF THE INVENTION

An object of the present invention is to provide a convenient supporting frame for mounting a one-way membrane inside a respiratory valve in order to solve such a technical problem that a one-way membrane in a respiratory valve cannot be replaced conveniently as the membrane is mounted on an existing support. The supporting frame is designed as an integral structure with a valve body. By means of the supporting frame, not only the one-way membrane can be conveniently mounted, but also a separate supporting frame is reduced, which makes an internal structure of the respiratory valve more compact and removes the risk that the supporting frame falls out.

To achieve the above object, the present invention provides a supporting frame for mounting a one-way membrane inside a respiratory valve. The supporting frame is provided on a radial cross-section of an air inlet channel in the respiratory valve and designed as an integral structure with the air inlet channel. A mounting hole for the one-way membrane to be mounted is provided at the center of the supporting frame, and an edge of the mounting hole is circumferentially distributed with several air holes.

As further improvement to the above technical solution, a notch for a cylindrical supporting structure in the one-way membrane to penetrate is provided at a side of the mounting hole that is close to a valve port of the respiratory valve.

The supporting frame for mounting a one-way membrane inside a respiratory valve according to the present invention has advantages in that:

by designing the supporting frame of the present invention as an integral structure with a valve body of the respiratory valve, fixing components needed by an existing support are simplified, so that an internal structure of the respiratory valve is made more compact, and the risk that the supporting frame falls out is removed; and by opening a notch on a mounting hole of the supporting frame, it is more convenient to replace the one-way membrane, increasing greatly a working efficiency.

### BRIEF DESCRIPTION OF THE DRAWINGS

FIG. 1 is a structural schematic diagram of mounting a one-way membrane with an existing support.
FIG. 2 is a side cross sectional view of fixing a support inside a respiratory valve with a sealing ring.
FIG. 3 is a side cross sectional view of fixing a support inside a respiratory valve with protrusions provided on an inner wall of an air inlet channel.
FIG. 4 is a side cross sectional view of a supporting frame provided inside a respiratory valve in accordance with the present invention.
FIG. 5 is a front view of a supporting frame provided inside a respiratory valve.

**Reference numerals**

| | | |
|---|---|---|
| 1. support | 101. mounting hole | 2. one-way membrane |
| 201. conic small tip | 202. conic large tip | 203. circular surface |
| 204. cylindrical supporting structure | 3. air inlet channel | 4. sealing ring |
| 5. protrusion | 6. valve body | 7. valve port |
| 8, supporting frame | 801. mounting hole | 802. air hole |
| 803. notch | | |

### DETAILED DESCRIPTION OF THE INVENTION

In conjunction with the accompanying drawings and embodiments, a supporting frame for mounting a one-way membrane inside a respiratory valve according to the present invention is illustrated in detail below.

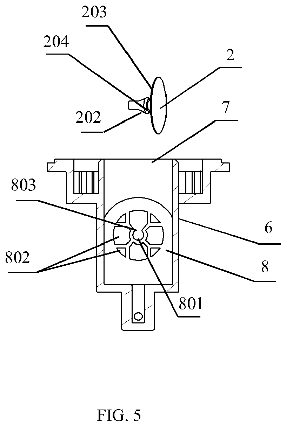

The present invention provides a supporting frame for mounting a one-way membrane inside a respiratory valve. As shown in Fig. 4, the supporting frame 8 is provided on a radial cross section of an air inlet channel 3 in the respiratory valve and designed as an integral structure with the air inlet channel 3. That is, the present invention forms the supporting frame 8 for mounting a one-way membrane 2 and a valve body 6 as an integral structure. As shown in Fig. 5, a mounting hole 801 for the one-way membrane 2 to be mounted is provided at the center of the supporting frame 8, an edge of the mounting hole 801 being circumferentially distributed with several air holes.

Based on the above structured supporting frame, as shown in Fig. 5, a notch 803 for a cylindrical supporting structure 204 in the one-way membrane 2 to penetrate is provided at a side of the mounting hole 801 that is close to a valve port 7 of the respiratory valve. In addition, several air holes 802 are circumferentially distributed along the center of the supporting frame 8.

As shown in Fig. 5, specific steps for mounting the one-way membrane 2 inside the respiratory valve with the supporting frame 8 are as below:

first of all, the respiratory valve is opened to expose the valve port 7, and a manner of mounting downward from the valve port 7 of the respiratory valve is employed. Since a size of the valve port 7 is smaller and not easy to mount the one-way membrane 2, the mounting hole 801 on the supporting frame 8 is designed as a hole of non-entire circle, and the notch 803 is provided at the top of the mounting hole 801, at which point one air hole 802 right above faces the notch 803 of the mounting hole 801.

Then, the cylindrical supporting structure 204 between a conic large tip 202 and a circular surface 203 of the one-way membrane 2 is aligned with the notch 803 and then pressed downward into the mounting hole 801. A diameter of the mounting hole 801 shall be slightly larger than that of the cylindrical supporting structure 204, and a size of the notch 803 may be determined in accordance with a diameter size of the cylindrical supporting structure 204 so as to guarantee a certain amount of compression of the cylindrical supporting structure 204 when being pressed into the notch 803.

When the one-way membrane 2 needs to be replaced, it only needs to pull up a conic tip of the one-way membrane 2 at the air inlet channel end, the cylindrical supporting structure 204 may slide out of the notch 803, thereby being easy for detachment.

Finally, it should be explained that the aforementioned embodiments are merely used for illustrating, rather than limiting the technical solutions of the present invention. Although the present invention has been described in detail with reference to the embodiments, those skilled in the art will understand that modifications or equivalent substitutions can be made to the technical solutions of the present invention without departing from the scope and spirit of the technical solutions of the present invention, and thereby should all be encompassed within the scope of the claims of the present invention.

## Claims

1. A supporting frame for mounting a one-way membrane inside a respiratory valve, **characterized in that** the supporting frame (8) is provided on a radial cross-section of an air inlet channel (3) in the respiratory valve and designed as an integral structure with the air inlet channel (3); and a mounting hole (801) for the one-way membrane (2) to be mounted is provided at the center of the supporting frame (8), an edge of the mounting hole (801) being circumferentially distributed with several air holes.

2. The supporting frame for mounting a one-way membrane inside a respiratory valve according to claim 1, **characterized in that** a notch (803) for a cylindrical supporting structure (204) in the one-way membrane (2) to penetrate is provided at a side of the mounting hole (801) that is close to a valve port (7) of the respiratory valve.
